# EUROPÄISCHE PATENTANMELDUNG

(11) **EP 1 036 784 A1**
(43) Veröffentlichungstag der Anmeldung: **20.09.2000**
(21) Anmeldenummer: 00101565.0
(22) Anmeldetag: 27.01.2000
(51) Int. Cl.: C07C 209/36, B01J 23/46, B01J 23/64, B01J 23/89

(54) **Verfahren zur katalytischen Hydrierung von Dinitrotoluol sowie Katalysator**

(30) Priorität: 17.03.1999 DE 19911865
(71) Anmelder: Degussa-Hüls Aktiengesellschaft, 60287 Frankfurt am Main (DE)
(72) Erfinder: Auer, Emmanuel, Dr., 60528 Frankfurt/Main (DE); Gross, Michael, 60385 Frankfurt/Main (DE); Packruhn, Uwe, 60389 Frankfurt/Main (DE)

(57) **Zusammenfassung**

Dinitrotoluol wird katalytisch hydriert, wobei als Katalysator ein multimetallischer Katalysator eingesetzt wird. Der Katalysator enthält Iridium sowie wenigstens ein Dotierungselement aus der Gruppe Vanadium, Nickel, Mangan, Eisen, Kobalt, Kupfer und/oder Platin.

## Beschreibung

Die Erfindung betrifft ein Verfahren zur katalytischen Hydrierung von Dinitrotoluol sowie einen Katalysator.

Der Einsatz von edelmetallhaltigen Katalysatoren für die katalytische Hydrierung von aromatischen Nitroverbindungen in flüssiger Phase ist seit langem bekannt (G.C. Bond, P.B. Walls, Advan. Catal. Relat. Subj. 15, 1964, 92)

Aromatische Amine stellen heute zentrale Bausteine in der Herstellung von Polymeren, gummitechnischen Erzeugnissen, Agro- und Pharmachemikalien dar. Insbesondere Toluoldiamin ist ein wichtiges Zwischenprodukt in der Synthese von Diisocyanaten, die als Monomere für die Herstellung von Polyurethanen in Form verschiedener Werkstoffen (Schäume, Elastomere) verwendet werden. Die katalytische Hydrierung des durch Nitrierung von Toluol gewonnenen Dinitrotoluols in Flüssigphase stellt dabei technisch die Hauptroute zur Synthese von Toluoldiamin dar.

Obwohl Palladium-Katalysatoren für die katalytischen Hydrierung von Dinitrotoluol (DNT) zu Toluoldiamin (TDA) in der industriellen Anwendung verbreitet sind, ist eines der zentralen Probleme die Deaktivierung des Katalysators durch die Bildung unerwünschter Nebenprodukte, wie z.B. ringhydrierte Derivate oder Dimerisations -/ Oligomersationsprodukte von partiell hydrierten Zwischenprodukten der Reaktion, die in der Literatur unter dem Begriff 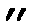 tar formation" beschrieben sind.

Bekannt ist daher eine Reihe von Publikationen, die sich mit besonderen Methoden, durch Modifikation der Palladiumkatalysatoren mit Eisen oder anderen Metallen die Selektivität in der Flüssigphasen-Hydrierung von DNT zu TDA zu erhöhen und gleichzeitig die Ausbeute an Toluoldiamin zu verbessern, beschäftigen.

Es ist bekannt, einen eisenmodifizierten Palladiumkatalysators auf einem hydrophoben Rußträger ( 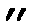 oleophilic carbon black") herzustellten (US 3 127 356). Insbesondere die Verwendung eines sehr feinteiligen Rußträgers (z.B. Acetylenrußes) und die Dotierung des Palladiums mit Elementen, wie Eisen und/oder Platin, führt bei dem bekannten Verfahren zu einer deutlichen Verbesserung der Aktivitätsrate (Umsatz Dinitrotoluol, bezogen auf eingesetztes Metall).

Bekannt ist die Verwendung von modifizierten Palladiumkatalysatoren (EP 002 308 B1). Dabei wird durch den Einsatz eines hochoberflächigen Aktivkohleträgers eine hohe Dispersion des Palladiums auf der Trägeroberfläche erreicht. Dies führt zu einer Verbesserung der Aktivität in der katalytischen Hydrierung von Dinitrotoluol, die in Methanol oder anderen geeigneten Solventien durchgeführt wird.

Die DE 196 36 214 A1 beschreibt die Verwendung geträgerter Iridiumkatalysatoren für die Hydrierung halogensubstituierter Amine. In diesem Fall war das Ziel, durch Verwendung der modifizierten Iridiumkatalysatoren die Dehalogenierung bei der katalytischen Hydrierung halogensubstituierter Nitroverbindungen zu unterdrücken.

Andere bekannte Flüssigphasenprozesse verwenden geträgerte Nickel- oder aktivierte Nickelkatalysatoren, die mit Elementen, wie Eisen, modifiziert sein können, um bessere Selektivitäten, d.h. geringere Nebenproduktbildung, zu gewährleisten und die Ausbeute an Toluoldiamin zu steigern (vgl. DE 4323 687).

Bekannt ist weiterhin Verwendung von geträgerten Palladiumkatalysatoren in einer Schmelze von Dinitrotoluol bei gleichzeitiger Entfernung des bei der Hydrierung gebildeten Wassers aus der Reaktionsmischung. Dabei soll ein intensiver Kontakt zwischen Katalysator und Substrat in Abwesenheit eines Lösungsmittels ermöglicht werden, um die Synthese von Toluoldiamin zu beschleunigen(US 2976 320).

Aus der JP-A-60-172-947 ist die Verwendung platinhaltiger oder palladiumhaltiger geträgerter Katalysatoren für die Flüssigphasen-Hydrierung von Dinitrotoluol bekannt. Dabei wird durch Verwendung hochaktiver Platinkatalysatoren und durch kontinuierliches Entfernen des gebildeten Reaktionswassers das chemische Gleichgewicht der Reaktion verschoben und das gewünschte Toluoldiamin in hohen Ausbeuten erhalten.

Die bekannten Verfahren der Flüssigphasen-Hydrierung von Dinitrotoluol zu Toluoldiamin weisen den Nachteil auf, daß bei Palladiumkatalysatoren oder modifizierten Palladium-und/oder Platinkatalysatoren durch die Bildung unerwünschter Nebenprodukte, wie zum Beispiel ringhydrierter Produkte, unvollständig hydrierter Zwischenstufen (Methylnitroanilin) oder insbesondere der Dimerisations- und Oligomerisationsprodukte ( 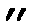 tar formation") die Selektivität der Reaktion abnimmt, und die genannten Nebenprodukte zu einer Deaktivierung der Katalysatoren verbunden mit Einbußen bei der Ausbeute an Toluoldiamin führen. Edelmetallhaltige Katalysatoren haben sich insbesondere bei höheren Drücken als weniger selektiv erwiesen.

Aufgabe der vorliegenden Erfindung ist es, durch Auswahl und Synthese eines geeigneten Hydrierkatalysators die Selektivität der katalytischen Hydrierung von Dinitrotoluol zu Diaminotoluol verbessern, die Bildung von Nebenprodukten zurückzudrängen und damit die Ausbeute an Toluoldiamin zu erhöhen.

Gegenstand der Erfindung ist ein Verfahren zu katalytischen Hydrierung von Dinitrotoluol zu Toluoldiamin, welches dadurch gekennzeichnet ist, daß man als Katalysator einen multimetallischen Katalysator, der neben Iridium wenigstens ein Metall aus der Gruppe Vanadium, Nickel, Mangan, Eisen, Kobalt, Kupfer und/oder Platin auf einem Träger enthält, verwendet.

Geeignete Träger für den Katalysator können Aktivkohle oder oxidische Materialien wie Aluminiumoxid, Siliciumdioxid, Titandioxid oder Mischoxide davon sein.

Zur Herstellung des erfindungsgemäßen Katalysators kann der Träger zunächst in Wasser suspendiert und durch die Zugabe der entsprechenden Salzlösungen imprägniert werden. Anschließend können die Metalle durch Zugabe geeigneter Basen in Form ihrer Hydroxide auf dem Träger aufgefällt und mit Hilfe eines wasserlöslichen Reduktionsmittels bei Temperaturen zwischen 0 und 100°C reduziert werden. Gegebenenfalls kann die Reduktion mit Wasserstoffgas in der Flüssigphase oder am getrockneten Katalysator erfolgen. Die Reihenfolge des Zusammenfügens von Trägermaterial, Wasser, Metallsalzlösungen und wasserlöslichem Reduktionsmittel kann auch variiert werden.

Die katalytische Hydrierung von Dinitrotoluol kann im diskontinuierlichen oder kontinuierlich betriebenen Rührreaktor in Gegenwart eines Lösungsmittels, wie zum Beispiel Methanol oder Toluol erfolgen. Sie kann auch in einem Gemisch aus Toluodiamin/Wasser insbesondere bei kontinuierlichen Verfahren erfolgen.

Die erfindungsgemäße Hydrierung von Dinitrotoluol zu Toluoldiamin kann bei Temperaturen zwischen 70 und 200°C, vorzugsweise 90 und 150°C, und Drücken zwischen 1 und 100 bar, vorzugsweise 10 bis 50 bar durchgeführt werden. Wird die Hydrierung kontinuierlich betrieben, so muß die Menge umgesetzten Eduktes durch Nachdosieren ersetzt und das Produkt/Wassergemisch aus dem Reaktor entfernt werden.

Die erfindungsgemäße Hydrierung von Dinitotoluol zu Toluoldiamin in Gegenwart des erfindungsgemäßen Katalysators zeichnet sich vorteilhafterweise vor allem durch eine niedrige Nebenproduktbildung und hohe Ausbeuten an Toluoldiamin aus. Die unerwünschte Bildung ringhydrierter Derivate und unvollständig hydrierter Intermediate wird nicht beobachtet. Auch die Di- und Oligomerisierung unterschiedlicher Reaktionszwischenstufen ( 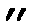 tar formation") ist deutlich geringer. Die Ausbeute an Toluoldiamin liegt in allen Fällen über denen, die unter Verwendung von bekannten geträgerter Palladium- oder modifizierter Palladiumkatalysatoren erzielt werden konnten.

Ein weiterer Gegenstand der Erfindung ist ein Katalysator, welcher dadurch gekennzeichnet ist, daß er ein multimetalischer Katalysator ist und neben Iridium wenigstens ein Metall aus der Gruppe Vanadium, Kupfer und/oder Platin auf einem Träger enthält. Der Träger kann Aktivkohle, Aluminiumoxid, Siliciumdioxid oder ein Mischoxid davon sein.

Der erfindungsgemäße Katalysator kann bei der Hydrierung von Dinitrotoluol zu Toluoldiamin eingesetzt werden.

### Beispiel 1

Es werden 94,85 g Aktivkohle in deionisiertem Wasser suspendiert. Zu dieser Suspension werden 21,74g Hexachloroiridium(IV)säure(23%ig) hinzugegeben. Nach Aufheizen auf 80°C und Einstellen des pH-Wertes auf 7 gibt man 0,34 g Ammoniumvanadat, gelöst in 100 ml Wasser, zur Suspension, reduziert mit Natriumhypophosphit und filtriert den Katalysator ab. Der fertige Katalysator enthält, bezogen auf das Gesamtgewicht, 5 Gew.-% Iridium und 0,15 Gew.-% Vanadium.

### Beispiel 2

Nach Suspendieren von 44,7 g Aktivkohle in deionisiertem Wasser gibt man 10,87 g Hexachloroiridium(IV)säure(23%ig) und 0,54 g Mangan-(II)-chlorid-Tetrahydrat zur Suspension hinzu. Man stellt den pH-Wert alkalisch (pH = 9), reduziert bei 80°C mit einer frisch bereiteten Natriumborhydridlösung (2 Gew.-%) und filtriert den Katalysator ab. Der fertige Katalysator enthält, bezogen auf das Gesamtgewicht, 5 Gew.-% Iridium und 0,3 Gew.-% Mangan.

### Beispiel 3

Man suspendiert 94,7 g Aktivkohle in deionisiertem Wasser und gibt zur Suspension Lösungen von 21,74 g Hexachloroiridium(IV)säure(23%ig)und 0,44g Eisen (III) chlorid, sowie 0,34 g Ammoniumvanadatlösung in 2,5 M Natronlauge hinzu. Man stellt die Suspension alkalisch (pH 10), reduziert mit frisch zubereiteter Natriumborhydridlösung (2 Gew.-%) und filtriert den Katalysator ab. Der fertige Katalysator enthält, bezogen auf das Gesamtgewicht, 5 Gew.-% Iridium, 0,15 Gew.-% Eisen und 0,15 Gew.-% Vanadium.

### Beispiel 4

In gleicher Weise wie in Beispiel 3 wird ein Katalysator unter Verwendung von 17,4 g Hexachloroiridium(IV)säurelösung (23%ig), 4,03 g Hexachloroplatin(IV)säurelösung(25%ig) und 0,73 g Eisen(III)chlorid hergestellt. Der fertige Katalysator enthält, bezogen auf sein Gesamtgewicht, 4 Gew.-% Iridium, 1 Gew.-% Platin und 0,5 Gew.-% Eisen.

### Beispiel 5

In Analogie zu Beispiel 1 der DE 196 36 214 wird ein Katalysator hergestellt, der bezogen auf sein Gesamtgewicht, 5 Gew.-% Iridium und, bezogen auf Iridium, 4,2 Gew.-% Mangan und 6,2 Gew.-% Eisen enthält.

### Beispiel 6

In Analogie zu Beispiel 3 der DE 196 36 214 wird ein Katalysator hergestellt, der bezogen auf sein Gesamtgewicht, 5 Gew.-% Iridium und, bezogen auf Iridium, 2,1 Gew.-% Cobalt und 8,4 Gew.-% Eisen enthält.

### Beispiel 7

In Analogie zu Beispiel 2 der DE 196 36 214 wird ein Katalysator hergestellt, der bezogen auf sein Gesamtgewicht, 5 Gew.-% Iridium und, bezogen auf Iridium, 10,5 Gew.-% Eisen enthält.

### Beispiel 8

In Analogie zu Beispiel 4 der DE 196 36 214 wird ein Katalysator hergestellt, der bezogen auf sein Gesamtgewicht, 5 Gew.-% Iridium und, bezogen auf Iridium, 6,3 Gew.-% Nickel und 4,2 Gew.-% Mangan enthält.

### Vergleichsbeispiel 1

In Analogie zu Beispiel 4 der US 3127 356 wird ein bimetallischer Palladium/Eisen Katalysator auf Shawinigan Black hergestellt.

### Vergleichsbeispiel 2

Als Vergleichskatalysator wird ein 5 Gew.-% beladenen Standard Palladiumkatalysator der Firma Degussa (E 101 R/W 5% Pd) eingesetzt.

### Anwendungsbeispiele

Die Katalysatoren gemäß den Beispielen 1 bis 8 sowie die Vergleichsbeispiele 1 und 2 werden hinsichtlich ihrer Aktivität und Selektivität in der diskontinuierlich durchgeführten Hydrierung von Dinitrotoluol zur Toluoldiamin getestet. Dabei werden die folgenden Reaktionsparameter eingehalten:
- Reaktionsdruck: 20 bar / 50 bar
- Temperatur: 100°C / 120°C
- Lösungsmittel: Methanol

Es werden jeweils 50 g Dinitrotoluol, gelöst in 200 ml Methanol, quantitativ hydriert. Der Endpunkt der Reaktion ist durch den rapiden Abfall der Wasserstoffaufnahme auf Null präzise bestimmbar. Es wird stets eine Katalysatormenge von 0,5 Gew.-%, bezogen auf eingesetztes Dinitrotoluol, verwendet. Die Nebenprodukte in dem Reaktionsprodukt werden anschließend mit GC (Gas-chromatographie) und HPLC bestimmt.

Die entstandenen Nebenprodukte werden in drei Gruppen eingeteilt:
- Nebenprodukt 1:: Ringhydrierte Folgeprodukte des Dinitrotoluol, Methylnitroanilin und Toluoldiamin
- Nebenprodukt 2:: Unvollständig hydrierte Verbindungen (zum Beispiel Methylnitroanilin)
- Nebenprodukt 3:: Dimere, Trimere, Oligomere ( 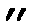 tar formation")

Die Proben werden nach einer Rührzeit von 15 Minuten nach dem Ende der Wasserstoffaufnahme aus dem Autoklaven entnommen.
Die Ergebnisse der Hydrierung bei 50 bar sind in der folgenden Tabelle zusammengefaßt.

| Katalysator nach Beispiel | Hydrierzeit (mm) | Ausbeute TDA (%) | Nebenprodukt 1 (%) | Nebenprodukt 2 (%) | Nebenprodukt 3 (%) |
|---|---|---|---|---|---|
| 1 | 55 | 99,3 | / | / | 0,7 |
| 2 | 45 | 99,1 | / | 0,1 | 0,8 |
| 3 | 45 | 99,2 | / | 0,1 | 0,8 |
| 4 | 40 | 99,0 | / | 0,4 | 0,6 |
| 5 | 50 | 99,3 | / | / | 0,7 |
| 6 | 45 | 99,3 | / | 0,1 | 0,6 |
| 7 | 60 | 98,9 | / | 0,2 | 0,9 |
| 8 | 60 | 99,0 | / | 0,2 | 0,8 |
| Vergleichs beispiel 1 | 40 | 98,3 | 0,3 | 0,3 | 1,0 |
| Vergleichsbeispiel 2 | 30 | 97,5 | 0,8 | 0,2 | 1,5 |

## Patentansprüche

1. Verfahren zu katalytischen Hydrierung von Dinitrotoluol zu Tolualdiamin, dadurch gekennzeichnet, daß man als Katalysator einen multimetallischen Katalysator, der neben Iridlum wenigstens ein Metall aus der Gruppe Vanadium, Nickel, Mangan, Eisen, Kobalt, Kupfer und/oder Platin auf einem Träger enthält, verwendet.

2. Verfahren nach Anspruch 1, dadurch gekennzeichnet, daß man als Träger Aktivkohle, Aluminiumoxid, Siliciumdioxid oder Mischoxid davon einsetzt.

3. Katalysator, dadurch gekennzeichnet, daß er ein multimetallischen Katalysator ist und neben Iridium wenigstens ein Metall aus der Gruppe Vanadium, Kupfer und/oder Platin auf einem Träger enthält.

4. Katalysator nach Anspruch 3, dadurch gekennzeichnet, daß der Träger Aktivkohle, Aluminiumoxid, Siliciumdioxid oder Mischoxid davon ist.

5. Verwendung des Katalysators nach Anspruch 4 bei Hydrierung von Dinitrotoluol zu Toluoldiamin.
